# EUROPEAN PATENT SPECIFICATION

(11) **EP 0 765 666 B1**
(45) Date of publication and mention of the grant of the patent: **06.02.2002**
(21) Application number: 96306279.9
(22) Date of filing: 29.08.1996
(51) Int. Cl.: A61K 31/7032, A61P 27/12

(54) **Use of oligosaccharides inhibiting Lewis-x glycolipid adhesion for the manufacture of a medicament for treating cataracts**
Verwendung von Oligosacchariden, welche die Adhäsion an Lewis-x Glycolipid hemmen, zur Herstellung eines Medikaments für die Behandlung von Katarakt
Utilisation d'oligosaccharides inhibant l'adhésion du glycolipides Lewis-X dans la fabrication d'un médicament déstiné au traitement de la cataracte

(30) Priority: 31.08.1995 JP 22418695
(43) Date of publication of application: 02.04.1997
(73) Proprietor: RESEARCH DEVELOPMENT CORPORATION OF JAPAN, Kawaguchi-shi, Saitama-ken (JP)
(72) Inventor: Ogiso, Manabu, Yokohama-shi, Kanagawa 232 (JP); Noro, Chikako, Tsukuba-shi, Ibaraki 305 (JP)
(74) Representative: Cockbain, Julian, Dr.

(56) References cited:
- EP-A- 0 120 328
- EP-A- 0 296 620
- WO-A-91/16320
- WO-A-92/19632
- US-A- 5 095 106
- DATABASE WPI Section Ch, Week 8925 Derwent Publications Ltd., London, GB; Class B05, AN 89-182758 XP002022458 & JP-A-01 121 215 (NIHON HAIPOKKUSU KK) , 12 May 1989
- BIOCHIM. BIOPHYS. ACTA, vol. 753, 1983, pages 89-96, XP000612288 TAO ET AL: "A new family of fucose containing gangliosides isolated from human senile cataracts"
- BIOCHIM. BIOPHYS. ACTA, vol. 1256, 17 May 1995, pages 166-74, XP000609574 OGISO ET AL: "IDENTIFICATION AND SYNTHETIC PATHWAY OF SIALYL-LEWISX-CONTAINING... "
- JOURNAL OF BIOLOGICAL CHEMISTRY, vol. 267, no. 10, 5 April 1992, pages 6467-6470, XP000609147 OGISO M ET AL: "SENILE CATARACT-RELATED ACCUMULATION OF LEWISX GLYCOLIPID IN HUMAN LENS*"
- BIOCHIM. BIOPHYS. ACTA, vol. 1315, no. 1, 17 January 1996, pages 29-36, XP000609569 MANABU OGISO ET AL: "IDENTIFICATION AND SYNTHETIC PATHWAY OF SIALYL-LEWISX-CONTAINING NEOLACTO-SERIES GANGLIOSIDES IN LENS TISSUES. 2. ENZYMATIC SYNTHESIS OF SIALYL-LEWISX GANGLIOSIDES IN MONKEY AND RAT LENSES"

## Description

The present invention relates to the use of agents in the manufacture of a medicament for inhibiting irregular binding of lens cells e.g. in the primate eye or especially in the human eye, caused by specific adhesion of Lewis^{x} glycolipids. More specifically, the present invention relates to a novel manner of effectively inhibiting the irregular binding of lens cells, this being a cause of human senile cataract, and an inhibitory agent for the irregular cell binding.

Cataract is a disease in which the lens within the eyeball becomes opaque, whereby the visual acuity is seriously impaired, and which finally causes loss of eyesight. Cataract may be caused congenitally or acquired. Known types of acquired cataract include senile, diabetic, traumatic and those caused by irradiation, such as ultraviolet or X-rays.

Since the recent improvement of living environments and nutritional conditions and the development of medical systems which improve life expectancy, senile cataract has become one of the typical senile diseases in many middle-aged and elderly patients, e.g. of more than fifty years of age.

The conventional practice of cataract therapy is a lens extirpation technique, in which cataractous lens is surgically removed from the eyeball. It is now possible to easily recover visual acuity after lens extirpation by inserting an intraoccular lens. In an ordinary lens extirpation, however, it is impossible to remove all lens cells from the eyeball, and some of the epithelial cells remain in the lens. Problems such as so-called after cataract, caused by these residual cells, can occur after such operations. In addition, there is a risk that a more serious disease such as uveitis or secondary glaucoma may develop.

While the lens extirpating operation is not always the best cataract therapy as described above, such a surgical operation is at present the only method to recover visual acuity impaired by cataract or to avoid the risk of loss of eyesight. No method of clearing a cataractous lens by administering a drug has yet been developed.

Development of an effective drug for prevention or therapy of cataract has not been successful mainly because the mechanism causing cataractous lens has not been clarified. More specifically, it is known that the lens is not governed by blood vessels or nerves unlike the other somatic cells and that the soluble proteins known as crystallins and the lamella structure of the cells keeps the lens transparent. Important features of the lens are that lens epithelial cells migrate into the lens nucleus to be accumulated during a lifetime, and that most of the cells have lost their nuclei. It is therefore difficult to conceive that, in spontaneous occurrence of a cataractous lens as in senile cataract, the cause should be expression of specific genes or degeneration of the cells themselves, and this makes it difficult to develop a drug through ordinary techniques.

The present inventors consider that the binding state of lens cells changes with ageing and this triggers senile cataract. The inventors carried out studies on glycolipid existing on cell membrane to clarify its role and changes, and have reported the following: the glycolipids of a human lens include those of the neolacto, globo, and ganglio series, and further, there are available Lewis^{x} glycolipid and sialyl-Lewis^{x} glycolipid known as cancer markers of somatic cells [see Ogiso et al. J. Biol. Chem. 267: 6467-6470 (1992); Ogiso et al. J. Biol. Chem. 268: 13242-13247 (1993); Ogiso, "Glycopathology" pages 299-304, Kodansha Scientific (1993); Ariga et al. J. Biol. Chem. 269: 2667-2675 (1994); Ogiso et al. Biochim. Biophys. Acta 1256: 166-174) (1995)].

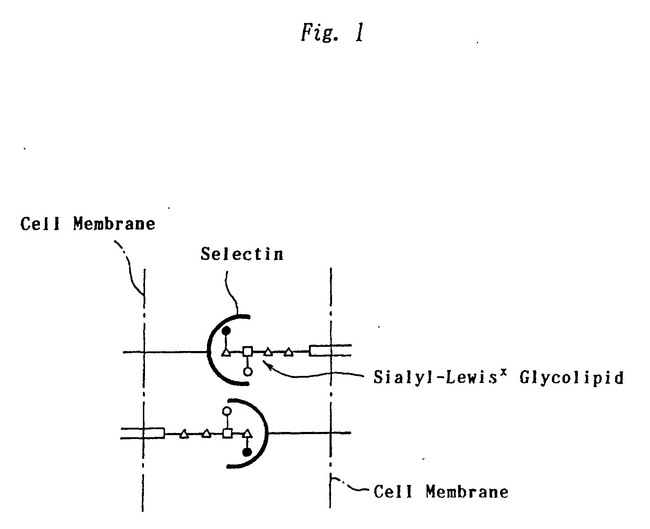

The inventors have also found that a protein, selectin, which binds specifically to sialyl-Lewis^{x} glycolipid in the presence of Ca²⁺, is present in the membrane of the lens cells, and that binding of selectin and sialyl-Lewis^{x} glycolipid forms regular combination of lens cells, maintaining transparency of the lens (see Fig. 1 of the accompanying drawings).

In human senile cataract, on the other hand, the content of ganglioside, which is an acidic glycolipid, increases with ageing and the progress of cataract [see Ogiso et al. Invest. Ophthalmol. Vis. Sci. 31: 2171-2179 (1990)]. There is Lewis^{x} antigen in neutral glycolipid, and the content thereof increases similarly with ageing and the progress of cataract [see Ogiso et al. J. Biol. Chem. 267: 6467-6470 (1992)]. However, the increase in the ganglioside content is dependent on ganglio-series glycolipid, and the increase in the content of sialyl-Lewis^{x} glycolipid brings about no marked change [see Ogiso et al. Exp. Eye. Res. 60: 317-323 (1995)].

Increase in the amount of Lewis^{x} glycolipid with ageing is therefore considered to result from removal of sialic acid from sialyl-Lewis^{x} glycolipid showing only a little change. More specifically, this terminal sialic acid is connected with galactose of the Lewis^{x} terminal, and ionized COO⁻ is present near the position of this connection. Binding of sialyl-Lewis^{x} glycolipid and selectin as described above is also considered to be reinforced by Ca²⁺ ions via this COO⁻. However, because this ionized COO⁻ is rather unstable in an aqueous solution, it is highly probable that removal of sialic acid occurs at this position in the absence of connection with selectin. Because of the absence of electric charge, Lewis^{x} glycolipid is considered to be relatively stable.

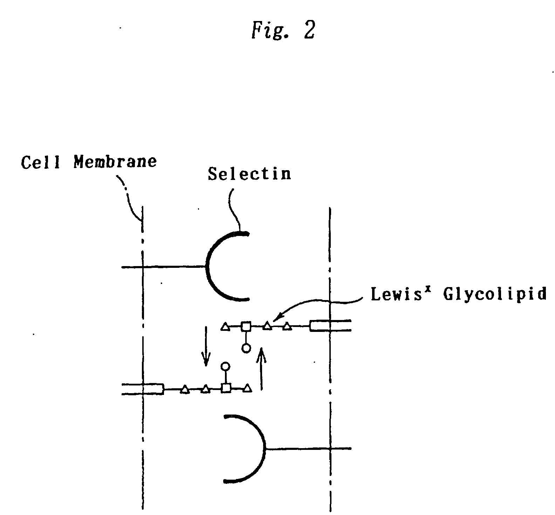

In a normal human lens, as described above, sialyl-Lewis^{x} glycolipid and selectin present on the surface of cells bind specifically to each other. With the progress of ageing, sialic acid is removed from sialyl-Lewis^{x} glycolipid and is transformed into Lewis^{x} glycolipid. The Lewis^{x} glycolipid is known to specifically combine with Lewis^{x} glycolipid on neighboring cell surfaces (see Fig. 2). This specific adhesion of Lewis^{x} glycolipids causes disturbance of regularity of lens cells binding, and this in turn exerts an effect on ion transport and transduction on the cell membrane, thus forming the primary cause of a cataractous lens, resulting in protein agglutination.

Furthermore, the present inventors clarified that the relationship between a cataractous lens and glycolipid as described above is specific to humans and monkeys (primates). More specifically, as a result of detection of expression of Lewis^{x} glycolipid and sialyl-Lewis^{x} glycolipid from the lenses of many mammals, expression of sialyl-Lewis^{x} glycolipid was observed in many mammals including rat, pig and primate, whereas it has been confirmed that expression of Lewis^{x} glycolipid in the lens is limited to primates [see Ogiso et al. Exp. Eye. Res. 59: 653-664 (1994)]. In fact, in a galactosemic cataract rat or an inherited cataractsuffering mouse, commonly used as cataract-model animals, there is not only no increase of Lewis^{x} glycolipids with the progress of cataract, but also synthesis of Lewis^{x} glycolipid does not intrinsically occur [see Ogiso et al. Exp. Eye. Res. 60: 317-323 (1995)]. These findings suggest that it is highly probable that human cataract is essentially different from that of laboratory animals, and the critical mechanism of lens cataract clarified by the use of laboratory animals will not basically agree with that of humans.

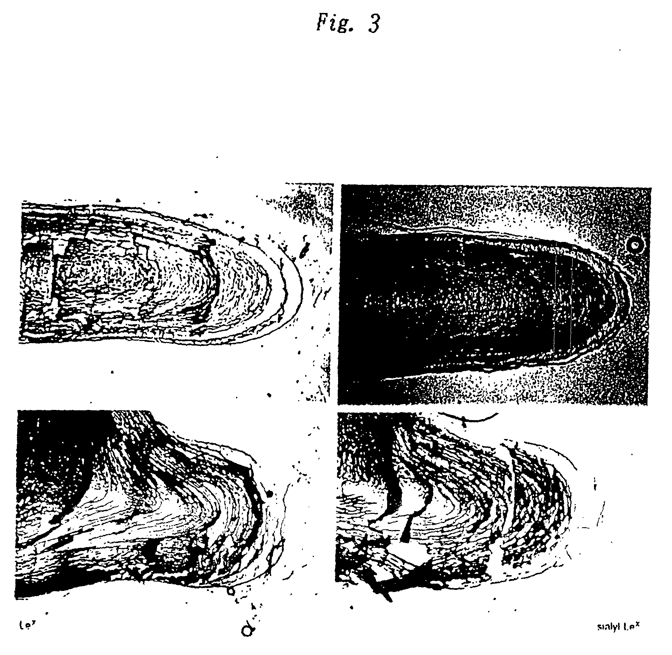

In primates, localization of Lewis^{x} glycolipid or sialyl-Lewis^{x} glycolipid is not detected in external epithelial cells, but in fiber cells in the lens [see Ogiso et al. Glycobiology, 4: 375-382 (1994); Ogiso et al. Exp. Eye. Res. 60: 317-323 (1995)]. This is confirmed by immunohistochemical identification of Lewis^{x} glycolipid in simian lens cells (see the photograph in Fig. 3 of the accompanying drawings), and from the fact that Lewis^{x} glycolipid (Le^{x}) and sialyl-Lewis^{x} glycolipid (sialyl-Le^{x}) were detected in the same fiber cell region in the lens.

Viewed from one aspect the invention thus provides the use of an oligosaccharide capable of binding to Lewis^{x} glycolipid which inhibits adhesion of Lewis^{x} glycolipid for the manufacture of a medicament for use in the treatment of the human, or non-human primate, eye to combat cataract.

The oligosaccharide used according to the invention inhibits adhesion of Le^{x} on lens cells by altering the amount of Le^{x} free to bind. This is effected by interfering with the ability of Le^{x} to bind to Le^{x}. This agent may thus for example be one which binds to Le^{x}.

The invention will now be described further by way of example and with reference to the accompanying drawings, in which:-
Fig. 1 is a schematic view illustrating cell binding for normal lens cells;
Fig. 2 is a schematic view illustrating cell binding of cataractous lens cells;
Fig. 3 is a photomicrograph of simian lens fiber cells showing the same localization of Lewis^{x} glycolipid and sialyl-Lewis^{x} glycolipid;

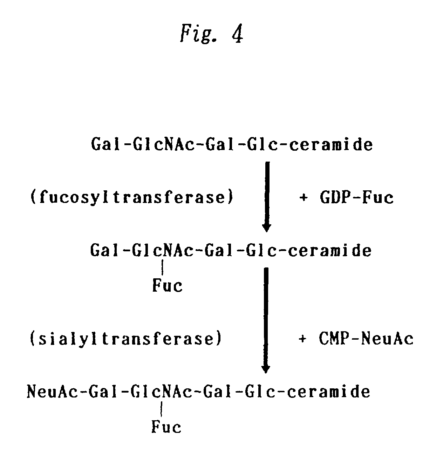

Fig. 4 illustrates a biosynthesis pathway from Lewis^{x} glycolipid to sialyl-Lewis^{x} glycolipid;

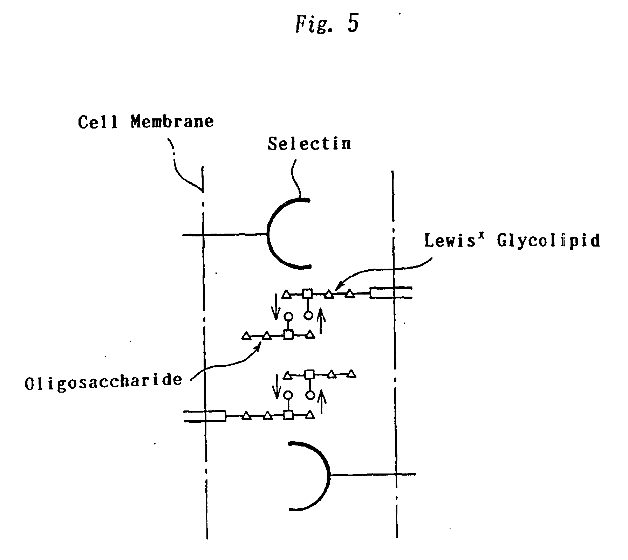

Fig. 5 is a schematic view illustrating the prevention of Lewis^{x} glycolipid binding using an oligosaccharide; and
Fig. 6 is a chemical formula illustrating the structure of Lewis^{x} glycolipid.

The present invention was developed on the basis of the following relationship between lens cells and glycolipid as found by the present inventors:
(1) In a normal lens, sialyl-Lewis^{x} glycolipid and selectin present on the fiber cell surface specifically bind each to other, and this maintains regular binding of the lens cells, making the lens transparent (see Fig. 1).
(2) With ageing, sialyl-Lewis^{x} glycolipids become desialated causing an increase in the Lewis^{x} glycolipids.
(3) The specific adhesion of Lewis^{x} glycolipids (see Fig. 2) results in an increased irregular binding between lens cells, which in turn cause cataract of the lens.

The basic principle of the present invention is therefore to prevent the specific adhesion of Lewis^{x} glycolipids as described in (3) above, and one of the ways of achieving this is to administer to the lens an oligosaccharide having a glycolipid sugar chain structure similar to that of Lewis^{x} glycolipid. More particularly, such an oligosaccharide specifically binds to Lewis^{x} glycolipid because of the affinity thereof with the glycolipid, thus effectively blocking the binding between Lewis^{x} glycolipids.

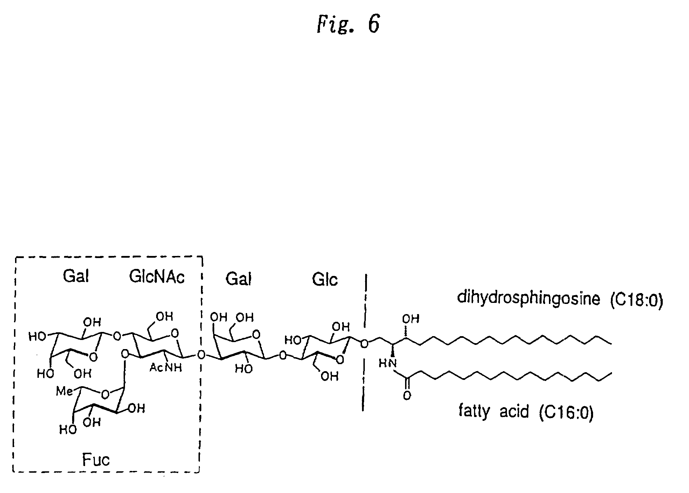

The oligosaccharide used in the present invention conveniently has a sugar chain having a structure identical with, or similar to, that of the sugar chain portion of Lewis^{x} glycolipid. In the structure of Lewis^{x} glycolipid shown in Fig. 6, at least the trisaccharide terminal portion (the portion inside the broken line in Fig. 6) can be used as a separate oligosaccharide. As such oligosaccharides, products and synthetic materials separated or extracted from human or cow's milk are commercially available, and can be employed for this purpose. For example, the products lacto-N-fucopentaose (LNFP III: made by Seikagaku Kogyo Company) and 3-fucosyllactose (3-FL: made by Seikagaku Kogyo Company), both oligosaccharides extracted from human milk, and the product Lewis^{x} type trisaccharide (made by Seikagaku Kogyo Company), a synthetic oligosaccharide, can be used.

The oligosaccharide can be formulated for administration for example as an eye-lotion. The content of each such Lewis^{x} glycolipid adhesion inhibiting agent may conveniently be within a range of from about 1 to 10 mg/ml.

A typical composition of an eye-lotion (aqueous solution) containing oligosaccharide is as follows:

| | |
|---|---|
| Boric acid | 1.60 g |
| Methyl cellulose | 0.50 g |
| Oligosaccharide (LNFP-III) | 0.1 to 1.0 g |
| Sterile water ad | 100 ml |

## Claims

1. The use of an oligosaccharide capable of binding to Lewis^{x} glycolipid which inhibits adhesion of Lewis^{x} glycolipid for the manufacture of a medicament for use in the treatment of the human or non-human primate eye to combat cataract.

2. Use as claimed in claim 1 wherein said oligosaccharide is separated or extracted from human milk.

3. Use as claimed in claim 1 wherein said oligosaccharide is separated or extracted from cow's milk.

4. Use as claimed in claim 2 wherein said oligosaccharide is lacto-N-fucopentaose or 3-fucosyllactose.

5. Use as claimed in claim 1 wherein said oligosaccharide is the synthetic oligosaccharide Lewis^{x} type trisaccharide.

## Patentansprüche

1. Verwendung eines Oligosaccharids, das dazu in der Lage ist, an ein Lewis^{x}-Glycolipid zu binden, und das die Adhäsion an einem Lewis^{x}-Glycolipid hemmt, für die Herstellung eines Medikaments zur Verwendung bei der Behandlung des Auges eines Menschen oder eines nicht-humanen Primaten, um einen Star bzw. eine Linsentrübung zu bekämpfen.

2. Verwendung nach Anspruch 1, wobei das Oligosaccharid aus Frauenmilch abgetrennt oder extrahiert ist.

3. Verwendung nach Anspruch 1, wobei das Oligosaccharid aus Kuhmilch abgetrennt oder extrahiert ist.

4. Verwendung nach Anspruch 2, wobei das Oligosaccharid Lacto-N-Fucopentaose oder 3-Fucosyllactose ist.

5. Verwendung nach Anspruch 1, wobei das Oligosaccharid das synthetische Oligosaccharid Lewis^{x}-Trisaccharid ist.

## Revendications

1. Utilisation d'un oligosaccharide capable de se lier au glycolipide de Lewis^{x}, qui inhibe l'adhésion du glycolipide de Lewis^{x}, pour la fabrication d'un médicament destiné à être utilisé dans le traitement de l'oeil de primate humain ou non humain pour combattre la cataracte.

2. Utilisation selon la revendication 1, dans laquelle ledit oligosaccharide est séparé ou extrait du lait humain.

3. Utilisation selon la revendication 1, dans laquelle ledit oligosaccharide est séparé ou extrait du lait de vache.

4. Utilisation selon la revendication 2, dans laquelle ledit oligosaccharide est le lacto-N-fucopentose ou le 3-fucosyllactose.

5. Utilisation selon la revendication 1, dans laquelle ledit oligosaccharide est l'oligosaccharide synthétique trisaccharide de type Lewis^{x}.
